# EUROPEAN PATENT APPLICATION

(11) **EP 1 813 923 A1**
(43) Date of publication of application: **01.08.2007**
(21) Application number: 07101263.7
(22) Date of filing: 26.01.2007
(51) Int. Cl.: G01K 1/14, G01K 13/00

(54) **An apparatus for the measurement of the temperature in a human body**

(30) Priority: 27.01.2006 IT TO20060054
(71) Applicant: Bergamin, Diego, 31050 Vedelago (IT); Vian, Alex, 30030 Robegano di Salzano (IT)
(72) Inventor: Bergamin, Diego, 31050 Vedelago (IT); Vian, Alex, 30030 Robegano di Salzano (IT)
(74) Representative: Jorio, Paolo

(57) **Abstract**

An apparatus (1) for the measurement of the temperature in a human body (2) comprising a garment (3) adapted to be worn by a subject subjected to the measurement of the body temperature; a temperature sensor (4), which is housed in the garment (3) so as to be arranged in contact with a predetermined area of the body surface of the subject when the garment (3) is worn by the subject himself/herself to provide a measurement signal related to the body temperature detected in the predetermined area; and a portable apparatus (5) separate from the garment (3), which is selectively adapted to receive the measurement signal from the temperature sensor (4) and is able to generate a message of the visual type indicating the measured body temperature.

## Description

The present invention relates to an apparatus for the measurement of the temperature in a human body.

As it is known, one of the instrumentations most commonly employed to measure the temperature in the human body is the mercury thermometer. Such a measurement instrumentation displays several drawbacks even though it has been widely used for a long time.

Firstly, in order to guarantee a reliable enough measurement of temperature, it is required that the subject subjected to the measurement remains in a partially motionless position for a prolonged time, typically a few minutes long, so as to ensure the maintenance of a contact of the thermometer on the body surface, so as to cause an expansion of the mercury, which is proportional to the measured body temperature.

As well as being inconvenient and uncomfortable, such a motionless condition is difficult to be obtained in all cases in which the measurement needs to be carried out on babies, children, elderly people, handicapped people, people with mental disorders or, more in general, in all those subjects having a partial or total difficulty to maintain a determined posture for a prolonged interval of time. Specifically, the movements carried out by such subjects during the measurement cause on one side interruptions of the contact or the resting of the thermometer on the body surface area, typically the axillary surface, and at the same time a displacement of the thermometer from the surface area itself thus causing a measurement which is poorly accurate.

The above described thermometers are also potentially dangerous for the subject subjected to the temperature measurement every time an accidental breaking of the glass bulb or tube containing the mercury occurs. Such a breaking may indeed cause on one side cuts or injuries on the body of the subject, and on the other side a leakage of the mercury from the receiving bulb, with obvious consequences on the health of the subject in the case of contact between the mercury itself and the body surface.

Therefore, it is the object of the present invention to implement an apparatus for the measurement of the temperature in the human body, being capable of measuring the body temperature in an almost instantaneous manner, being particularly accurate and practical to be used and avoiding at the same time any potentially dangerous or hazardous situation for the subject himself/herself.

According to the present invention, there is implemented an apparatus for the measurement of the temperature in a human body, as described in claim 1 and, preferably, in any of the following claims directly or indirectly dependent from claim 1.

According to the present invention there is also implemented a garment, as described in claim 15 and, preferably, in any of the following claims directly or indirectly dependent from claim 15.

The present invention will now be described with reference to the accompanying drawings, which show a non-limitative example of embodiment thereof, in which:
- figure 1 shows an apparatus for the measurement of the temperature in the human body, implemented according to the dictates of the present invention;
- figure 2 is a block diagram of a portable apparatus included in the apparatus for the measurement of the temperature in the human body shown in figure 1;
- figure 3 shows a variant of the apparatus for the measurement of the temperature in the human body shown in figure 1; and
- figure 4 is a diagram block of a portable apparatus included in the apparatus for the measurement of the temperature in the human body shown in figure 3.

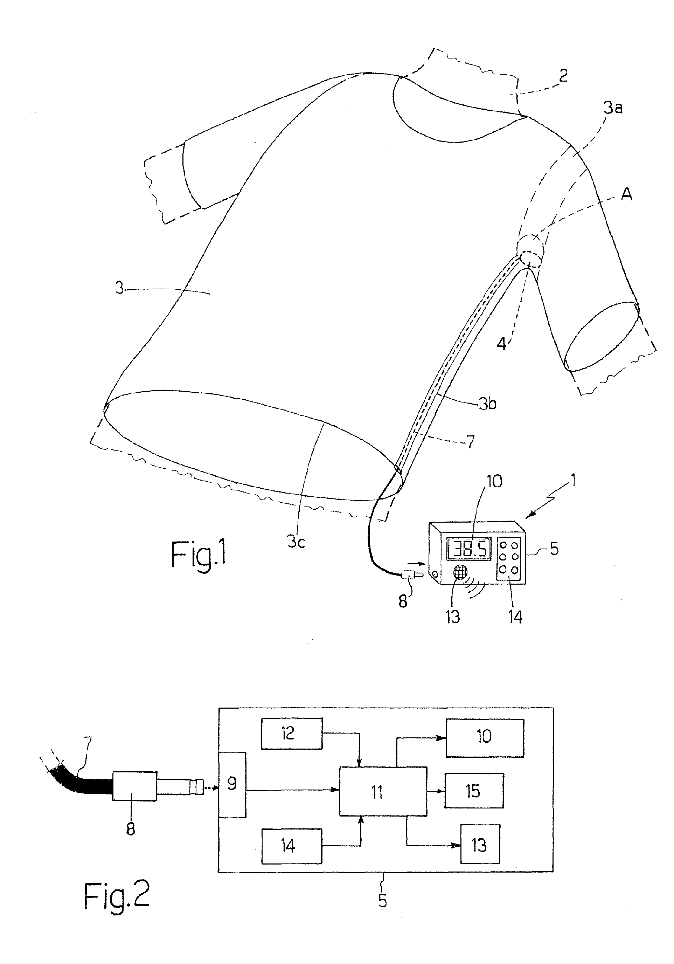

With reference to figure 1, numeral 1 indicates, as a whole, an apparatus for the instant measurement of the temperature in the body 2 of a human subject.

The apparatus 1 substantially comprises at least one garment 3 adapted to be worn by the subject subjected to the measurement of the temperature; at least one temperature sensor 4, which is permanently fixed to the garment 3 so as to be arranged in contact with a body surface zone or area A of the subject to provide a measurement signal related to the detected body temperature; and a portable apparatus 5 separate and independent of the garment 3, which is adapted to communicate with the temperature sensor 4 to receive an input measurement signal, and is able to generate a message of the visual type and, preferably but not necessarily, a message of the vocal type indicating the body temperature measured by the sensor 4 itself.

In the example shown in figure 1, the garment 3 comprises a shirt made of an elastic fabric of the known type capable of adhering the internal surface of the shirt to the body surface of the subject wearing it, while the sensor 4 is integrated in the shirt at the armhole portion 3a (indicated with a broken line in figures 1 and 2) so as to be arranged in contact with the axillary surface of an arm of the subject when the shirt is worn by the same.

According to a different embodiment, the shirt is made of an elastic fabric only at the armhole portion 3a, i.e. in the portion of the shirt surrounding the shoulder of the subject so as to advantageously ensure the maintenance of the contact between the sensor 4 and the body surface, independently of the posture taken by the subject himself/herself. The remaining part of the shirt may instead be made of any fabric.

As far as the sensor 4 is concerned, it may comprise a thermistor or any other similar electric "contact" transducer, which, when placed in contact with the body, is capable of generating in an almost instantaneous manner a measurement signal proportional to the body temperature of the same.

The sensor 4 may be housed in a seat or in a closed pocket (not shown) obtained in the shirt, or it may be permanently fixed in the internal part of the shirt itself so as to be arranged in direct contact with the body surface.

In the example shown in figure 1, the sensor 4 is adapted to be connected/disconnected to/from the portable apparatus 5 by means of a communication wire 7, which is permanently fixed on the shirt, and in its free end is provided with a connector 8 adapted to be connected/disconnected to/from the portable apparatus 5.

The communication wire 7 extends along the shirt from the armhole portion 3a along a side edge 3b existing on the side of the shirt and ends at the lower edge 3c of the shirt itself. Specifically, the side edge 3b may preferably correspond to a side stitching segment of the shirt, and the communication wire 7 may be housed, i.e. integrated within the stitching segment itself so as not to result in direct contact with the body 2 of the subject.

In this case, in the example shown in figure 1, the communication wire 7 partially protrudes from the lower portion 3c of the shirt with its free end (on which the connector 8 is connected), so as to allow the connection between the connector 8 and the portable apparatus 5.

With reference to figures 1 and 2, the portable apparatus 5 substantially comprises an interface 9 comprising a connection module which is coupleable to the connector 8 to receive the measurement signal; a display device 10, such as e.g. a display; and an electronic control circuit 11 capable of receiving the measurement signal from the interface 9 and controlling the display of the measured temperature by means of the display.

The portable apparatus 5 may further comprise a power supply module 12, e.g. a battery adapted to feed power to the circuits of the portable apparatus 5; preferably but not necessarily, a vocal signalling module 13 adapted to generate the vocal message indicating the measured temperature; and preferably but not necessarily, a control panel 14 through which the user may control the activation/deactivation (on/off) of the measurement of the temperature.

The portable apparatus 5 finally comprises a communication module 15, which is adapted to activate a "long distance" communication through a communication network (not shown) with a remote processing unit (not shown) to transmit the measurement signal to the latter.

In this case, in the example shown in figure 2, the communication module 15 consists of a telephone modem adapted to communicate with the remote processing unit by implementing a determined telephone protocol operating according to ISDN or GSM, GPRS, UMTS standards or the like etc. It is apparent that the communication network may operate the exchange of information relative to the measured body temperature between the portable apparatus 5 and the remote processing unit by also implementing protocols other than those indicated above.

During use, the garment 3 is worn by the subject 2 such that the sensor 4 is appropriately placed in contact with the body surface of the same. At this point, the sensor 4 may be connected through the connector 8 to the portable apparatus 5, which is responsible for receiving and processing the measurement signal so as to instantly communicate the measured temperature in a visual manner and, preferably but not necessarily, by means of a vocal message.

The above described apparatus 1 displays the following advantages. Firstly, the garment 3 guarantees the maintenance of a correct contact position of the sensor 4 on the body surface, independently of the posture taken by the subject subjected to the measurement, who is free to carry out any movement during the measurement. Therefore, the apparatus is particularly effective, practical, and accurate in the measurement of the body temperature of babies, children, elderly people, handicap people, people with mental disorders or, more in general, in all subjects having a partial or total difficulty to take a determined posture for a prolonged period of time.

Furthermore, the apparatus 1 is simple and cost-effective to implement and is extremely safe because it is free from parts or components which may be dangerous for the health of the patient.

According to a different embodiment (not shown), the apparatus 1 comprises a plurality of garments 3, which are wearable by a plurality of subjects subjected to the measurement of the temperature, and are each provided with a temperature sensor 4 connectable, through the corresponding communication wire 7, to the portable apparatus 5 to provide the latter with a measurement signal.

The interface 9 of the portable apparatus 5 is connectable to a plurality of connectors 8 and is capable of handling a plurality of measurement signals, while the electronic control circuit 11, on the basis of a series of commands which may be given by the user by means of the control panel 14 is capable of activating the display device 10, and the vocal signalling module 13 to communicate the measured temperatures. The above described apparatus 1 may have an advantageous application in the hospital field in which it is required to carry out the measurement of the temperature on a plurality of patients. In this case, indeed, the apparatus 1 allows to instantly measure the body temperatures of an extremely high number of patients thus drastically decreasing the times typically required for the operators to carry out such a measurement operation.

It must be added that the presence of the communication module 15 in the portable apparatus 5 further allows the measurement of a plurality of temperatures by a remote processing unit which may be capable of carrying out the storage of the measured temperatures in each patient and maintaining at the same time the remote monitoring of its temperature. In detail, the remote processing unit may conveniently be capable of updating in a completely automatic manner the medical record (filed e.g. in an electronic format within the processing unit itself) containing the data corresponding to the temperatures acquired by the patient in different predetermined time intervals.

According to a different embodiment (not shown), the garment comprises a bandage or belt made of an elastic material, which houses the temperature sensor 4 therein and is adapted, during use, to be fitted on the arm of the subject so as to be positioned at the shoulder so that the sensor 4 is substantially maintained in contact with the axillary area.

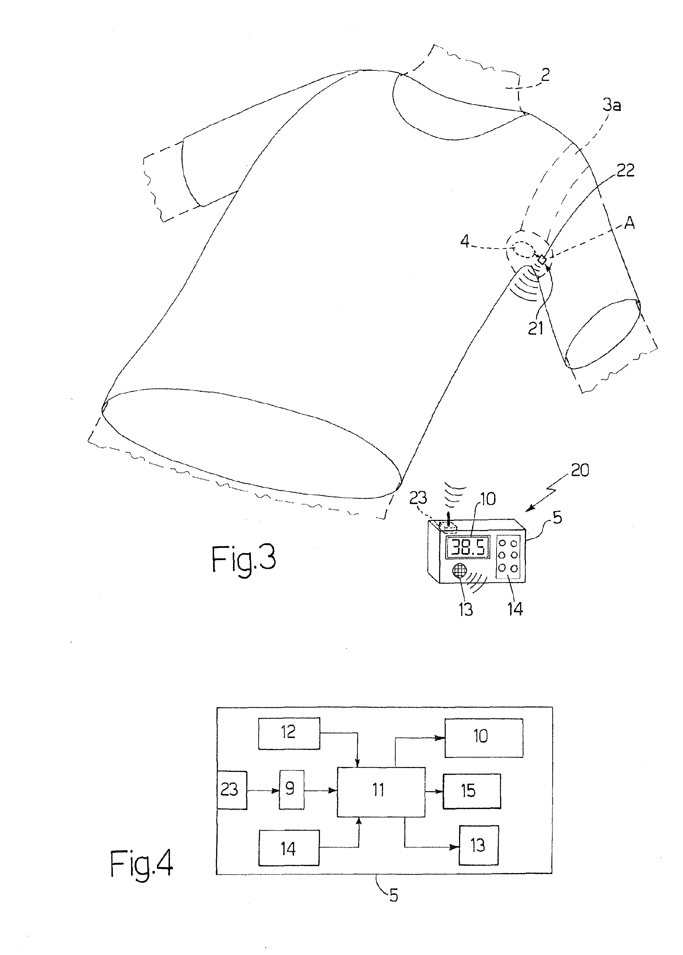

The embodiment shown in figures 3 and 4 relates to an apparatus 20, which is similar to apparatus 1, the component parts of which will be marked, where possible, with the same reference numbers marking corresponding parts of the apparatus 1.

The apparatus 20 is capable of performing a wireless communication between the temperature sensor 4 and the portable apparatus 5, thus advantageously eliminating the presence of the communication wire 7 from the garment 3.

For this purpose, the apparatus 20 differs from the apparatus 1 because it is free from communication wire 7 and is provided with a wireless communication system 21 comprising a transmission module 22 housed in the garment 3 and connected to the sensor 4, which is adapted to transmit the measurement signal to the portable apparatus 5; and a receiving module 23, included in the portable apparatus 5 (figure 4) to receive the measurement signal transmitted by the transmission module 22.

Specifically, in the example shown, the transmission module 22 is housed in the garment 3 at the temperature sensor 4, while the receiving module 23 is housed in the portable apparatus 5 and is connected to the interface 9 to provide it with the received input measurement signal (figure 4).

The communication between the modules 22 and 23 may be an infrared or radio frequency communication or any other type of wireless communication of the known type. In the case the communication is carried out by means of a wireless radio frequency system, the modules 22 and 23 may each be provided with an antenna for the transmission and reception of the signals.

The apparatus 20 displays the great advantage of eliminating the presence of the communication wire 7, thus determining a greater convenience in use.

According to a different embodiment (not shown), the apparatus 20 comprises a plurality of garments 3, which are wearable by a series of subjects subjected to the measurement of the temperature, and are each provided with a temperature sensor 4 adapted to communicate the measurement signal to the portable apparatus 5 by means of a wireless communication system.

The receiving module 23 of the portable apparatus 5 is capable of handling the reception of the measurement signals from the transmission modules 22 to appropriately provide them to the electronic control circuit 11 which controls the communication of the visual and/or vocal message indicating the measured temperatures. The portable apparatus 5 is therefore capable of selectively displaying or communicating by means of a visual or vocal message one or more measured temperatures.

## Claims

1. An apparatus (1) (20) for the measurement of the temperature in a human body (2) **characterised in that** it comprises at least one garment (3) adapted to be worn by a subject subjected to the measurement of the body temperature; at least one temperature sensor (4), which is internally fixed to said garment (3) so as to be arranged in contact with a predetermined body surface area (A) of the subject when said garment (3) is worn by the subject himself/herself, to provide a measurement signal related to the body temperature detected in said predetermined body surface area (A); and a portable apparatus (5) separate from the garment (3), which is adapted to receive the measurement signal from the temperature sensor (4) and is able to generate a visual message indicating the measured body temperature.

2. An apparatus (1) (20) according to claim 1, **characterised in that** said portable apparatus (5) is able to generate a message of the vocal type indicating the measured body temperature.

3. An apparatus (1) (20) according to claims 1 and 2, **characterised in that** said garment (3) is made at least partially of an elastic fabric at the portion of the garment (3) housing said at least one temperature sensor (4).

4. An apparatus (1) (20) according to any of the preceding claims, **characterised in that** said at least one sensor (4) is integrated in said garment (3) in a position such that it is arranged in contact with the external surface of an arm of the subject, when the garment (3) is worn by the subject himself/herself.

5. An apparatus (1) according to any of the preceding claims, **characterised in that** it comprises electric connection means (7, 8), which are adapted to allow the electric connection/disconnection between said temperature sensor (4) and said portable apparatus (5).

6. An apparatus (1) according to claim 5, **characterised in that** said electric connection means (7, 8) comprise at least one communication wire (7), which is integrated in said garment (3) and is provided in its free end with connector means (8) adapted to be connected/disconnected to said portable apparatus (5).

7. An apparatus (20) according to any of claims from 1 to 4, **characterised in that** it comprises communication means (21) adapted to implement a wireless communication between said temperature sensor (4) and said portable apparatus (5) to transmit said measurement signal to said portable apparatus (5).

8. An apparatus (20) according to claim 7, **characterised in that** said communication means (21) are adapted to reciprocally communicate by radio frequency.

9. An apparatus (20) according to claim 8, **characterised in that** said communication means (21) comprise transmitting means (22) housed internally to said garment (3) and adapted to transmit said measurement signal; and receiving means (23), which are housed in said portable apparatus (5) and are adapted to receive said measurement signal.

10. An apparatus according to any of the preceding claims, **characterised in that** said garment comprises a shirt or a bandage.

11. An apparatus according to any of the preceding claims, **characterised in that** said portable apparatus (5) comprises at least one display device (10) adapted to display the measured body temperature.

12. An apparatus according to any of claims from 2 to 10, **characterised in that** said portable apparatus (5) comprises at least one vocal signalling module (13) adapted to generate a vocal message indicating the measured body temperature.

13. An apparatus according to any of the preceding claims, **characterised in that** it comprises a plurality of garments (3) adapted to be worn by corresponding subjects each subjected to the measurement of his/her own body temperature; and a plurality of temperature sensors (4) permanently fixed to the corresponding garments (3) and adapted to provide said portable apparatus (5) with a plurality of measurement signals related to the corresponding measured body temperatures.

14. An apparatus according to any of the preceding claims, **characterised in that** said portable apparatus (5) comprises communication means (15) adapted to activate a long distance communication with a remote processing unit to transmit the measurement signal to said remote processing unit.

15. A garment (3) **characterised in that** it comprises at least one temperature sensor (4), which is permanently fixed to said garment (3) so as to be arranged in contact with a predetermined body surface area of a subject wearing said garment (3), and is adapted to provide a measurement signal related to the body temperature detected in said predetermined body surface area (A) to a portable apparatus (5), which is able to generate a visual and/or vocal message indicating the measured body temperature.

16. A garment according to claim 15, **characterised in that** it comprises a shirt at least partially made of an elastic fabric so as to adhere to the body of the subject wearing it; said temperature sensor (4) being integrated in the shirt at the armhole portion (3a) so as to be arranged in contact with the surface of an arm of the subject, when said shirt is worn by the same.

17. A garment (3) according to claim 15, **characterised in that** it comprises a shirt provided with an elastic fabric bandage arranged at the armhole portion (3a) and housing said temperature sensor (4).

18. A garment according to claim 15, **characterised in that** it comprises a bandage adapted to be fitted on the body of said subject and housing said temperature sensor (4).

19. A garment according to any of claims from 15 to 18, **characterised in that** it comprises electric connection means (7, 8), which are adapted to allow the electric connection/disconnection between said temperature sensor (4) and said portable apparatus (5).

20. A garment according to claim 19, **characterised in that** said electric connection means (7, 8) comprise at least one communication wire (7), which is integrated in said garment (3) and is provided in its free end with connector means (8) adapted to be connected/disconnected to/from said portable apparatus (5).

21. A garment according to any of claims from 15 to 18, **characterised in that** it comprises transmission means (22) adapted to transmit said measurement signal to said portable apparatus (5).
